# EUROPEAN PATENT SPECIFICATION

(11) **EP 2 452 714 B1**
(45) Date of publication and mention of the grant of the patent: **23.01.2013**
(21) Application number: 10014431.0
(22) Date of filing: 10.11.2010
(51) Int. Cl.: A61M 16/04

(54) **Tracheostomy tube connector**
Interner Tracheostomietubus
Tube de trachéostomie de type interne

(43) Date of publication of application: 16.05.2012
(73) Proprietor: Vitaltec Corporation, Taichung Hsien (TW)
(72) Inventor: Chiu, Sheng-Yu, Taichung Hsien (TW); Chang, Ti-Li, Taichung Hsien (TW)
(74) Representative: Hauck Patent- und Rechtsanwälte

(56) References cited:
- EP-A1- 1 803 478
- DE-A1- 3 223 592
- US-A- 5 067 496
- US-A1- 2002 029 782
- US-A1- 2008 041 391
- US-A1- 2009 095 302
- US-A1- 2009 235 936

## Description

### 1. Field of the Invention

The present invention relates to an inner type tracheostomy tube, and more particularly to an inner type tracheostomy tube that can easily and safely change an inner cannual of the inner type tracheostomy tube.

### 2. Description of Related Art

A patient who has dyspnea due to trachea obstruction or disease caused by weakness or drug effects may require a conventional tracheostomy tube inserted into the trachea through a stoma formed through the trachea to provide air from a respiratory tube directly to the patient through the conventional tracheostomy tube.

When changing the conventional tracheostomy tube, a surgical Operation is needed to inconveniently operate on the patient under a general anesthesia. A conventional inner type tracheostomy tube can be used to overcome the aforementioned problem and has an outer tube and an inner cannula. The inner cannula is connected to the outer tube by screwing or clamping. Thus, the inner cannula of the conventional inner type tracheostomy tube can be changed from the outer tube to improve the convenience of using the conventional tracheostomy tube.

From EP 1 803 478 A1, US 2009/095302 A1 and US 2009/235936 A1 a tracheotomy tube is known that includes an outer cannula having a fenestration and an inflatable cuff formed by a sleeve. The inner cannula can be releasably fixed to the outer cannula by locking tabs in engagement with couplers. The locking tabs are flexibly formed to the inner cannula, the couplers are provided at the proximal end of the outer cannula.

US 2002/029782 A1 describes a tracheotomy tube with an inner and an outer cannula. Unwanted separation of the inner cannula from the outer cannula can be achieved by installing a retaining ring which prevents the inner cannula from unwittingly being unlatched from the outer cannula. The retaining ring allows the air supply elbow to be separated which, in turn, permits the sensory alarms to properly sound when a disconnection of the air supply arises.

In US 5/067,496 a tracheostomy tube with an outer cannula and an inner cannula is disclosed. The tracheostomy tube comprises an annular retaining collar, having a flange with an aperture in longitudinal alignment is adapted to receive the inflation tube and is attached to the proximal end of the outer cannula. The inner cannula is secured to the outer cannula by a coupling connector having arms which engage the annular collar.

From US 2008/041391 A1 a coupling is known for connecting an air supply to a respiratory support device having a latching mechanism which prevents the coupling from inadvertently axially displacing from the respiratory support device. Non-axial forces are used to disengage the coupling from the respiratory support device.

DE 32 23 592 A1 describes an adjustable pipe clamp which is used for attachment of an attachment flange to a tracheostomy tube. A deformable sleeve is provided through whose hole the catheter is guided. The attachment flange can fixedly attached to tracheostomy tube via a clamping strip which can be tightend around the deformable sleeve.

However, the inner cannula of the conventional inner type tracheostomy tube is screwed with the outer tube and may be separated from the outer tube when the inner cannula is rotated with a respiratory tube and this is dangerous in use. Furthermore, when the inner cannula of the conventional inner type tracheostomy tube is connected to the outer tube by an exerted clamping structure, dirt may be accumulated on the exerted clamping structure causing infection. In addition, the patent may knock the exerted clamping structure of the inner cannula to cause the inner cannula to separate from the outer tube and this is unsafe in use.

To overcome the shortcomings, the present invention tends to provide an inner type tracheostomy tube to mitigate or obviate the aforementioned problems.

The main objective of the present invention is to provide an inner type tracheostomy tube that can easily and safely change an inner cannual of the inner type tracheostomy tube.

The inner type tracheostomy tube in accordance with the present invention has an outer tube and an inner cannula. The outer tube has an airway tube, an inflatable cuff, an inflating tube, a neck mount and a holding mount. The inflatable cuff is mounted around an inner end of the airway tube. The inflating tube is connected to and communicates with the inflatable cuff. The neck mount is securely mounted around an outer end of the airway tube. The holding mount is formed on the neck mount around the outer end of the airway tube and has a holding ring and two holding arms. The holding ring is formed on the neck mount. The holding arms are formed oppositely on the holding ring and protrude from the holding ring and each holding arm has a holding recess. The inner cannula is detachably connected to the outer tube and has an inner tube, a disk segment, two locking tabs, a connecting mount and a connector. The disk segment is formed on the inner tube and abuts the holding ring of the holding mount. The locking tabs are formed on the disk segment, are respectively connected to the holding arms and each locking tab has a shape corresponding to the holding recess, a locking portion and a pressing button. The connecting mount is securely mounted around the disk segment. The connector is securely mounted around the connecting mount.

Other objectives, advantages and novel features of the invention will become more apparent from the following detailed description when taken in conjunction with the accompanying drawings.

### IN THE DRAWINGS:

Fig. 1 is a perspective view of an inner type tracheostomy tube in accordance with the present invention;
Fig. 2 is an exploded perspective view of the inner type tracheostomy tube in Fig. 1;
Fig. 3 is an enlarged exploded perspective view of the inner type tracheostomy tube in Fig. 1;
Fig. 4 is an enlarged cross sectional side view of the inner type tracheostomy tube in Fig. 1;
Fig. 5 is a side view of the inner type tracheostomy tube in Fig. 1; and
Fig. 6 is an optional side view of an inner cannula of the inner type tracheostomy tube in Fig. 1.

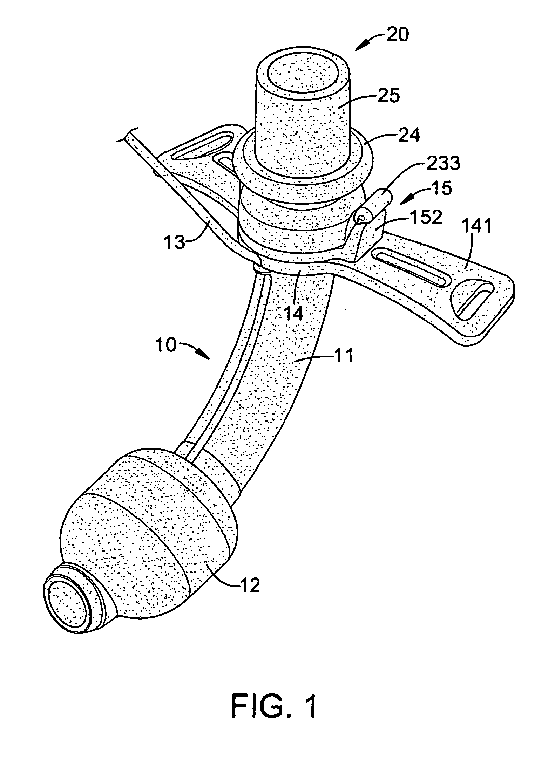

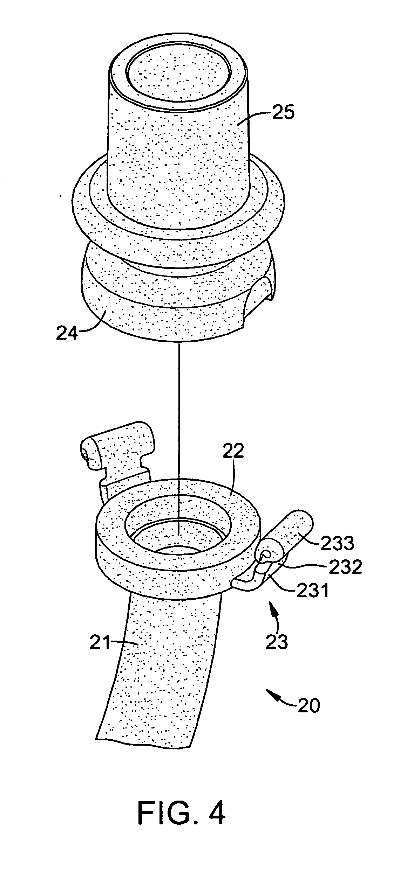

With reference to Figs. 1 to 5, an inner type tracheostomy tube in accordance with the present invention has an outer tube 10 and an inner cannula 20.

The outer tube 10 has an airway tube 11, an inflatable cuff 12, an inflating tube 13, a neck mount 14 and a holding mount 15.

The airway tube 11 may be a hard tube and has an inner end, an outer end and an external surface. The inflatable cuff 12 is mounted around the external surface near the inner end of the airway tube 11 and is inflated to form a seal in a patient's neck for mechanical breathing and need to be totally deflated when insert into or withdraw from patient's trachea. The inflating tube 13 is connected to and communicates with the inflatable cuff 12 to inflate or deflate the inflatable cuff 12.

The neck mount 14 may be an annular hard board, is securely mounted around the external surface of the airway tube 11 near the outer end of the airway tube 11 and has a top, two sides and two neck plates 141. The neck plates 141 are elongated and are respectively formed on and protrude from the sides of the neck mount 14 to allow a cord or strap to be connected to the neck plates 141 and are mounted around the patient's neck to hold the neck plates 141 in place and hold the outer tube 10 securely in the neck of the patient.

The holding mount 15 is formed on the neck mount 14 around the outer end of the airway tube 11 and has a holding ring 151 and two holding arms 152. The holding ring 151 is formed on the top of the neck mount 14 around the outer end of the airway tube 11 and has an external surface and a top. The holding arms 152 are formed oppositely on the external surface of the holding ring 151 and protrude from the top of the holding ring 151, and each holding arm 152 has a bottom, a top, an inner face, a holding recess 153 and an opening 154. The bottoms of the holding arms 152 are formed on the external surface of the holding ring 151 and respectively abut the neck plates 141 of the neck mount 14. The tops of the holding arms 152 extend above the top of the holding ring 151.

The holding recesses 153 may be rectangular and are respectively formed in the inner faces of the holding arms 152 between the tops of the holding ring 151 and the holding arms 152, and each holding recess 153 has a width. The openings 154 are respectively formed through the tops of the holding arms 152 and respectively communicate with the holding recesses 153 of the holding arms 152, and each opening 154 has a width smaller than the width of the holding recess 153.

The inner cannula 20 is detachably connected to the outer tube 10 and has an inner tube 21, a disk segment 22, two locking tabs 23, a connecting mount 24 and a connector 25.

The inner tube 21 is mounted in the airway tube 11 of the outer tube 10 and has an inner end, an outer end and an external surface. The inner end of the inner tube 21 is mounted in the airway tube 11 near the inner end of the airway tube 11. The outer end of the inner tube 21 extends out of the holding ring 151 of the holding mount 15. The disk segment 22 is formed on the external surface of the inner tube 21 at the outer end of the inner tube 21, abuts the holding ring 151 of the holding mount 15 and has a bottom, a top and an external surface. The bottom of the disk segment 22 abuts the top of the holding ring 151.

The locking tabs 23 are formed on the external surface of the disk segment 22 from the bottom of the disk segment 22 and protrude from the top of the disk segment 22 and are respectively connected to the holding arms 152 of the holding mount 15. Each locking tab 23 has a shape corresponding to the holding recess 153, a lower end, an upper end, a middle, a locking portion 231, a neck portion 232 and a pressing button 233. The locking portions 231 are respectively formed on the lower ends of the locking tabs 23, are formed on the external surface of the disk segment 22 and are respectively and securely mounted in the holding recesses 153 of the holding arms 152, and each locking portion 231 has a width. The neck portions 232 are respectively formed on the middles of the locking tabs 23, are respectively connected with the locking portions 231 and extend out of the openings 154 of the holding arms 152. Each neck portion 232 has a width smaller than the width of the locking portion 231. The pressing buttons 233 are respectively formed on the upper ends of the locking tabs 23 and are respectively connected with the neck portions 232 above the openings 154 of the holding arms 152.

The connecting mount 24 may be soft, is securely mounted around the disk segment 22 between the locking tabs 23 and has a bottom end and a top end. The bottom end of the connecting mount 24 is securely mounted around the disk segment 22 between the locking tabs 23. The connector 25 is securely mounted around the top end of the connecting mount 24.

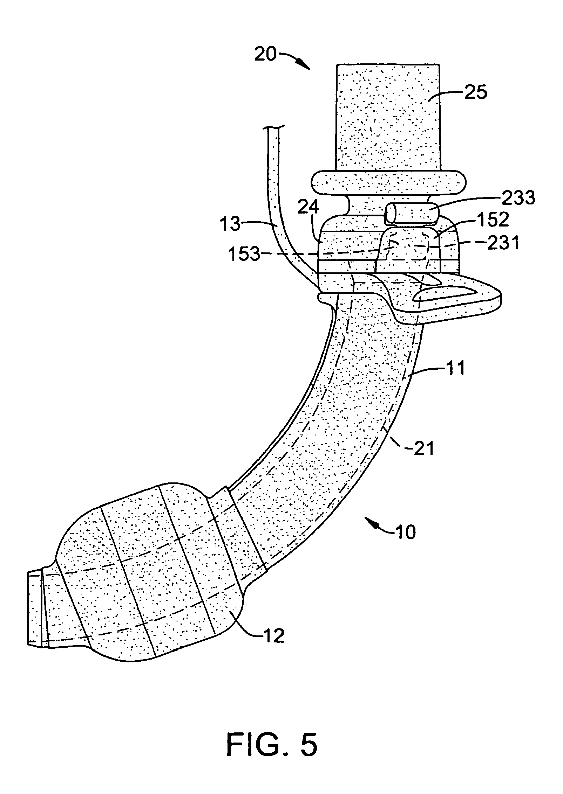

With reference to Figs. 1 and 5, the airway tube 11 of the outer tube 10 is inserted into a patient's trachea through a stoma defined in the trachea by a surgical operation. A cord or strap can be mounted around the patient's neck to hold the neck plates 141 in place and the inflatable cuff 12 is inflated through the inflating tube 13 and abuts against an inner surface of the patient's trachea to hold the outer tube 10 securely in the neck of the patient. The connector 25 of the inner cannula 20 is connected to a ventilator or a respiratory tube, so that air can be forced directly into the trachea of the patient through the outer tube 10 and the inner cannula 20 of the inner type tracheostomy tube in accordance with the present invention.

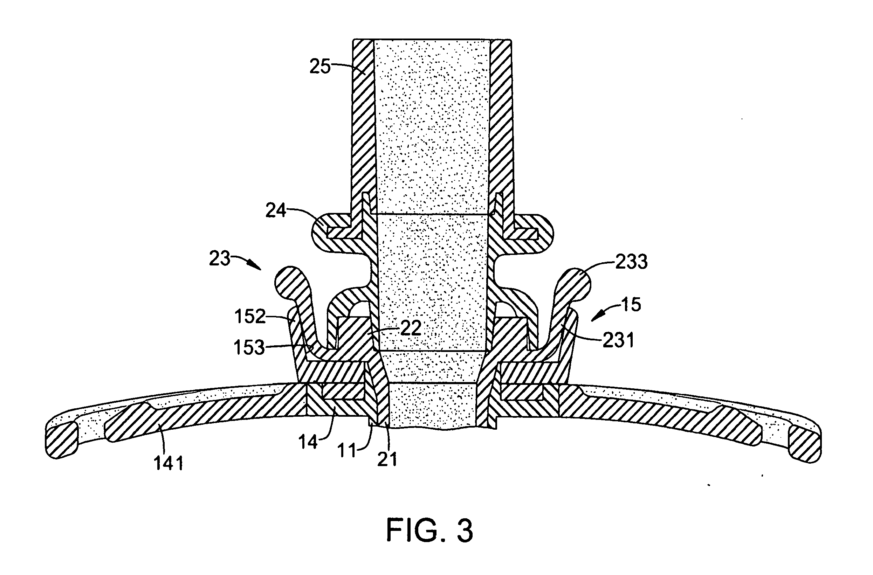

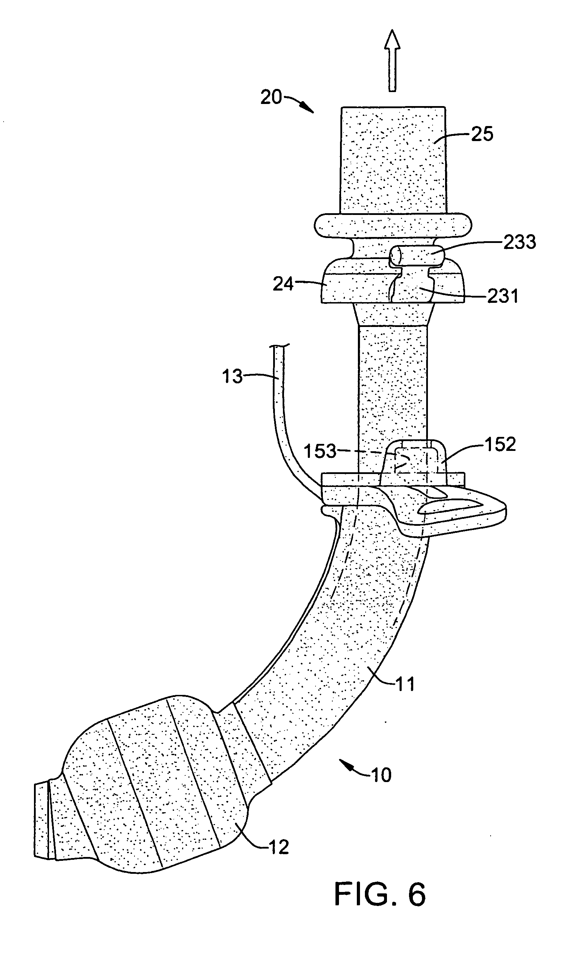

With reference to Figs. 3 to 6, when the inner cannula 20 is needed to change, the pressing buttons 233 of the locking tabs 23 are pressed to separate the locking portions 231 of the locking tabs 23 respectively from the holding recesses 153 of the holding arms 152. Then, the inner tube 21 of the inner cannula 20 can be pulled out of the airway tube 11 of the outer tube 10 by pulling the pressing buttons 233 of the locking tabs 23 and a new inner cannula 20 can be inserted and held with the outer tube 10. Accordingly, to change an inner cannula 20 with a new one is convenient and easy.

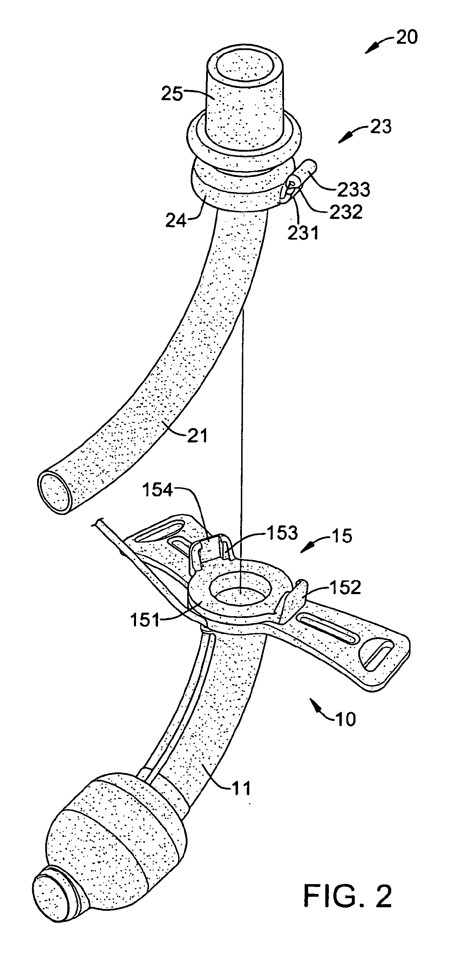

With further reference to Fig. 2, the locking portions 231 of the locking tabs 23 are respectively and securely mounted in the holding recesses 153 of the holding arms 152 and this can make the holding arms 152 mounting around the locking tabs 23 prevent dirt from accumulating on the locking tabs 23 and to avoid the patient knocking the locking tabs 23 of the inner cannula 20 to separate the inner cannula 20 from the outer tube 10 and this is clean and safe to use.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An inner type tracheostomy tube, comprising
• an outer tube (10) having an airway tube (11), an inflatable cuff (12), an inflating tube (13), a neck mount (14) and a holding mount (15), said airway tube (11) having an inner end, an outer end and an external surface, said inflatable cuff (12) being mounted around the external surface near the inner end of the airway tube (11), said inflating tube (13) being connected to and in communication with the inflatable cuff (12) to inflate or deflate the inflatable cuff (12), said neck mount (14) being securely mounted around the external surface of the airway tube (11) near the outer end of the airway tube (11), said neck mount (14) having a top, and two sides, said holding mount (15) having a holding ring (151) and two holding arms (152), said holding ring (151) being formed on the top of the neck mount (14) around the outer end of the airway tube (11) wherein said holding ring (151) has an external surface and a top, said two holding arms (152) being formed oppositely on the external surface of the holding ring (151) and protruding from the top of the holding ring (151), each of said holding arms (152) having an inner face, a bottom formed on the external surface of the holding ring (151), a top extending above the top of the holding ring (151), and a holding recess (153) formed in the inner face of the holding arm (152) between the tops of the holding ring (151) and the holding arm (152), said holding recess (153) having a shape and a width,
• an inner cannula (20) being detachably connected to the outer tube (10) having an inner tube (21), two locking tabs (23), a connecting mount (24) and a connector (25), said inner tube (21) being mounted in the airway tube (11) of the outer tube (10), wherein said inner tube (21) has an external surface, an inner end mounted in the airway tube (11) near the inner end of the airway tube (11) and an outer end extending out of the holding ring (151) of the holding mount (15), wherein each of said locking tabs (23) has a lower end, an upper end and a middle, and each of said locking tabs (23) having a shape corresponding to that of the holding recess (153) in a corresponding holding arm (152),
**characterized in that**
• said holding mount (15) is formed on the neck mount (14) around the outer end of the airway tube (11),
• said inner cannula (20) comprises a disk segment (22) being formed on the external surface of the inner tube (21) at the outer end of the inner tube (21), abutting the holding ring (151) of the holding mount (15), wherein said disk segment (22) has an external surface, a bottom abutting the top of the holding ring (151) and a top,
• said two locking tabs (23) are formed on the external surface of the disk segment (22) from the bottom of the disk segment (22) and protruding from the top of the disk segment (22) and respectively connected to the holding arms (152) of the holding mount (15),
• each of said locking tabs (23) further has a locking portion (231) formed on the lower end of the locking tab (23), formed on the external surface of the disk segment (22) and securely mounted in the holding recess (153) of the corresponding holding arm (152), said locking portion (231) having a width, each of said locking tabs (23) further has a pressing button (233) formed on the upper end of the locking tab (23) above the corresponding holding arm (152),
• said connecting mount (24) is securely mounted around the disk segment (22) between the locking tabs (23), wherein said connecting mount (24) has a bottom end securely mounted around the disk segment (22) between the locking tabs (23) and a top end,
• said connector (25) is securely mounted around the top end of the connecting mount (24).

2. The inner type tracheostomy tube as claimed in claim 1, wherein
each holding recess (153) is rectangular;
each holding arm (152) has an opening (154) formed through the top of the holding arm (152), communicating with the holding recess (153) of the holding arm (152) and having a width smaller than the width of the holding recess (153) of the holding arm (152);
each locking tab (23) has a neck portion (232) formed on the middle of the locking tab (23), is connected with the locking portion (231) of the locking tab (23) and extending out of the opening (154) of the corresponding holding arm (152) and having a width smaller than the width of the locking portion (231) of the locking tab (23); and
each pressing button (233) is connected with a corresponding neck portion (232) above the opening (154) of a corresponding holding arm (152).

3. The inner type tracheostomy tube as claimed in claim 1 or 2, wherein
the neck mount (14) is an annular hard board and has two neck plates (141) being elongated and respectively formed on and protruding from the sides of the neck mount (14); and
the bottoms of the holding arms (152) respectively abut the neck plates (141) of the neck mount (14).

## Patentansprüche

1. Ein innerartiger Tracheostoma, das umfasst
• Einen äußeren Tubus (10), der einen Luftwegschlauch, eine aufblasbare Manschette, einen aufblasbaren Tubus (13), eine Halshalterung (14) und eine Festhaltehalterung (15) aufweist, wobei der genannte Luftwegschlauch (11) ein inneres Ende, ein äußeres Ende und eine externe Oberfläche aufweist, die genannte aufblasbare Manschette (12) um die externe Oberfläche in der Nähe des inneren Endes des Luftwegschlauches (11) montiert ist, der genannte aufblasbare Tubus (13) verbunden und in Kommunikation mit der aufblasbaren Manschette (12) ist, um die aufblasbare Manschette aufzublasen oder Luft abzulassen, die genannte Halshalterung (14) sicher um die äußere Oberfläche des Luftwegschlauchs (11) nahe dem äußeren Ende des Luftwegschlauchs (11) befestigt ist, die genannte Halshalterung (14) eine Oberseite und zwei Seiten aufweist, die genannte Festhaltehalterung (15) einen Haltering (151) und zwei Haltearme (152) aufweist, der genannte Haltering (151) an der Oberseite der Halshalterung (14) um das äußere Ende des Luftwegschlauches (11) geformt ist, wobei der genannte Haltering (151) eine externe Oberfläche und eine Oberseite aufweist, die genannten Haltearme (152) an der externen Oberfläche des Halterings (151) gegenüberliegend geformt sind und sich von der Oberseite des Halterings (151) erstrecken, jeder der genannten Haltearme (152) eine innere Fläche, einen an der externen Fläche des Halterings (151) gebildeten Boden, eine sich über die Oberseite des Halterings (151) erstreckende Oberseite und eine in der inneren Fläche des Haltearms (152) gebildete Halteausnehmung (153) zwischen den Oberseiten des Halterings (151) und dem Haltearm (152) aufweist, die genannte Halteausnehmung (153) eine Form und eine Breite aufweist,
• eine innere Kanüle (20), die lösbar mit dem äußeren Tubus (10) verbunden ist, der einen inneren Tubus (21), zwei Verriegelungshebel (23), eine Verbindungshalterung (24) und einen Verbinder (25) aufweist, der genannte innere Tubus (21) in dem Luftschlauch (11) des äußeren Tubus (10) montiert ist, wobei der genannte innere Tubus (21) eine äußere Oberfläche, ein in den Luftwegschlauch (11) nahe dem inneren Ende des Luftwegschlauches (11) montiertes inneres Ende und ein sich aus dem Haltering (151) der Festhaltehalterung (15) erstreckendes äußeres Ende aufweist, wobei jeder der Verriegelungshebel (23) ein unteres Ende, ein oberes Ende und eine Mitte aufweist und jeder der genannten Verriegelungshebel (23) eine zu der Halteausnehmung (153) korrespondierende Form in einem korrespondierendem Haltearm (152) aufweist, **dadurch gekennzeichnet, dass**
• Die genannte Festhaltehalterung (15) an der Halshalterung (14) um das äußere Ende des Luftwegschlauches (11) gebildet ist,
• Die genannte innere Kanüle (20) ein Scheibensegment (22) umfasst, das an der externen Oberfläche des inneren Tubus (21) an dem äußeren Ende des inneren Tubus (21), das an den Haltering (151) der Festhaltehalterung (15) angrenzt, gebildet ist, wobei das genannte Scheibensegment (22) eine externe Oberfläche, ein an der Oberseite des Halterings (151) angrenzenden Boden und einer Oberseite aufweist,
• Die zwei genannten Verriegelungshebel (23) an der äußeren Oberfläche des Scheibensegments (22) vom Boden des Scheibensegments (22) ausgebildet sind und von der Oberseite des Scheibenelements (22) hervorstehen bzw. mit den Haltearmen (152) der Festhaltehalterung (15) verbunden sind,
• Jeder der genannten Verriegelungshebel (23) weitere an dem unteren Ende des Verriegelungshebels (23) gebildete Verriegelungsstücke (231) hat, die an der externen Oberfläche des Scheibensegments (22) gebildet sind und sicher in der Halteausnehmung (153) des korrespondierenden Haltearms (152) montiert sind, das genannte Verriegelungsstück (231) eine Breite hat, jeder der genannten Verriegelungshebel (23) weiter eine Druckknopf (233) aufweist, der an dem oberen Ende des Verriegelungshebels (23) über dem korrespondierenden Haltearm (152) gebildet ist,
• Die genannte Verbindungshalterung (24) ist sicher um das Scheibensegment (22) zwischen den Verriegelungshebeln (23) montiert, wobei die genannte Verbindungshalterung (24) ein Bodenende, das sicher um das Scheibensegment (22) zwischen den Verriegelungshebeln (23) montiert ist, und einem Oberseitenende aufweist,
• Der genannten Verbinder (25) ist sicher um das Oberseitenende der Vernimdungshalterung (24) montiert.

2. Innenartiger Tracheostoma nach Anspruch 1, wobei jede Halteausnehmung (153) rechteckig ist; jeder Haltearm (152) ein durch die Oberseite des Haltearms geformte Öffnung aufweist, die mit der Halteausnehmung (153) des Haltearms (152) kommuniziert und eine kleinere Breite als die Breite der Halteausnehmung (153) des Haltearms (152) aufweist,
jeder Verriegelungshebel (23) ein an der Mitte des Verriegelungshebels (23) geformtes Halsstück (232) aufweist und mit dem Verriegelungsstück (231) des Verriegelungshebels (23) verbunden ist und sich aus der Öffnung (154) des korrespondierenden Haltearmes (152) erstreckt und eine kleinere Breite als die Breite des Verriegelungsstückes (132) des Verriegelungshebels (23) aufweist; und
jeder Druckknopf (233) mit einem korrespondierenden Halsstück (232) über der Öffnung (154) eines korrespondierenden Haltearms (152) verbunden ist.

3. Innerartiger Tracheostoma nach Anspruch 1 oder 2, wobei die Halshalterung (14) ein ringförmiges hartes Brett und zwei Halsplatten (141) aufweist, die länglich bzw. auf und hervorstehend von den Seiten der Halshalterung gebildet sind, bzw. die Böden der Hatearme (152) an die Halsplatten (141) der Halshalterung angrenzen.

## Revendications

1. Tube de trachéostomie de type interne, comprenant
• un tube extérieur (10) ayant un tube des voies aériennes (11), une manchette gonflable (12), un tube de gonflage (13), une monture de cou (14) et une monture de retenue (15), ledit tube des voies aériennes (11) ayant une extrémité intérieure, une extrémité extérieure et une surface externe, ladite manchette gonflable (12) étant montée autour de la surface externe près de l'extrémité intérieure du tube des voies aériennes (11), ledit tube de gonflage (13) étant raccordé à la manchette gonflable (12) et étant en communication avec elle pour gonfler ou dégonfler la manchette gonflable (12), ladite monture de cou (14) étant montée solidement autour de la surface externe du tube des voies aériennes (11) près de l'extrémité extérieure du tube des voies aériennes (11), ladite monture de cou (14) ayant un sommet, et deux côtés, ladite monture de retenue (15) ayant une bague de retenue (151) et deux bras de retenue (152), ladite bague de retenue (151) étant formée au sommet de la monture de cou (14) autour de l'extrémité extérieure du tube des voies aériennes (11), ladite bague de retenue (151) ayant une surface externe et un sommet, lesdits deux bras de retenue (152) étant formés de façon opposée sur la surface externe de la bague de retenue (151) et dépassant du sommet de la bague de retenue (151), chacun desdits bras de retenue (152) ayant une face intérieure, un fond formé sur la surface externe de la bague de retenue (151), un sommet s'étendant au-dessus du sommet de la bague de retenue (151), et un creux de retenue (153) formé dans la face intérieure du bras de retenue (152) entre les sommets de la bague de retenue (151) et le bras de retenue (152), ledit creux de retenue (153) ayant une forme et une largeur,
• une canule intérieure (20) étant raccordée de façon détachable au tube extérieur (10) ayant un tube intérieur (21), deux languettes de blocage (23), une monture de raccordement (24) et un connecteur (25), ledit tube intérieur (21) étant monté dans le tube des voies aériennes (11) du tube extérieur (10), ledit tube intérieur (21) ayant une surface externe, une extrémité intérieure montée dans le tube des voies aériennes (11) près de l'extrémité intérieure du tube des voies aériennes (11) et une extrémité extérieure s'étendant hors de la bague de retenue (151) de la monture de retenue (15), chacune desdites languettes de blocage (23) ayant une extrémité inférieure, une extrémité supérieure et un milieu, et chacune desdites languettes de blocage (23) ayant une forme correspondant à celle du creux de retenue (153) dans un bras de retenue (152) correspondant,
**caractérisé en ce que**
• ladite monture de retenue (15) est formée sur la monture de cou (14) autour de l'extrémité extérieure du tube des voies aériennes (11),
• ladite canule intérieure (20) comprend un segment de disque (22) qui est formé sur la surface externe du tube intérieur (21) à l'extrémité extérieure du tube intérieur (21), venant buter contre la bague de retenue (151) de la monture de retenue (15), ledit segment de disque (22) ayant une surface externe, un fond venant buter contre le sommet de la bague de retenue (151) et un sommet,
• lesdites deux languettes de blocage (23) sont formées sur la surface externe du segment de disque (22) à partir du fond du segment de disque (22) et en dépassant du sommet du segment de disque (22) et respectivement raccordées aux bras de retenue (152) de la monture de retenue (15),
• chacune desdites languettes de blocage (23) a en outre une portion de blocage (231) formée sur l'extrémité inférieure de la languette de blocage (23), formée sur la surface externe du segment de disque (22) et montée solidement dans le creux de retenue (153) du bras de retenue (152) correspondant, ladite portion de blocage (231) ayant une largeur, chacune desdites languettes de blocage (23) a en outre un bouton de pression (233) formé sur l'extrémité supérieure de la languette de blocage (23) au-dessus du bras de retenue (152) correspondant,
• ladite monture de raccordement (24) est montée solidement autour du segment de disque (22) entre les languettes de blocage (23), ladite monture de raccordement (24) ayant une extrémité de fond montée solidement autour du segment de disque (22) entre les languettes de blocage (23) et une extrémité supérieure,
• ledit connecteur (25) est monté solidement autour de l'extrémité supérieure de la monture de raccordement (24).

2. Tube de trachéostomie de type interne selon la revendication 1, dans lequel
chaque creux de retenue (153) est rectangulaire ;
chaque bras de retenue (152) a une ouverture (154) formée à travers le sommet du bras de retenue (152), communiquant avec le creux de retenue (153) du bras de retenue (152) et ayant une largeur inférieure à la largeur du creux de retenue (153) du bras de retenue (152);
chaque languette de blocage (23) a une portion de cou (232) formée sur le milieu de la languette de blocage (23), est raccordée à la portion de blocage (231) de la languette de blocage (23) et s'étend hors de l'ouverture (154) du bras de retenue (152) correspondant et a une largeur inférieure à la largeur de la portion de blocage (231) de la languette de blocage (23) ; et
chaque bouton de pression (233) est raccordé à une portion de cou (232) correspondante au-dessus de l'ouverture (154) d'un bras de retenue (152) correspondant.

3. Tube de trachéostomie de type interne selon la revendication 1 ou 2, dans lequel
la monture de cou (14) est une plaque dure annulaire et a deux plateaux de cou (141) allongés et formés respectivement sur les côtés de la monture de cou (14) et dépassant de ces derniers ; et
les fonds des bras de retenue (152) viennent respectivement buter contre les plateaux de cou (141) de la monture de cou (14).
